# FASCICULE DE BREVET EUROPEEN

(11) **EP 1 348 408 B1**
(45) Date de publication et mention de la délivrance du brevet: **29.09.2010**
(21) Numéro de dépôt: 03011733.7
(22) Date de dépôt: 11.12.1998
(51) Int. Cl.: A61F 2/38

(54) **Endoprothèses de l'articulation du genou**
Kniegelenkendoprothesen
Knee joint endoprostheses

(30) Priorité: 12.12.1997 FR 9716044
(43) Date de publication de la demande: 01.10.2003
(62) Demande divisionnaire de: 98959968.3
(73) Titulaire: TORNIER, 38330 Saint-Ismier (FR)
(72) Inventeur: Tornier, Alain, 38330 Saint Ismier (FR)
(74) Mandataire: Myon, Gérard Jean-Pierre

(56) Documents cités:
- EP-A- 0 346 183
- US-A- 5 358 527
- US-A- 5 658 342

## Description

La présente invention est relative à des perfectionnements apportés aux prothèses totales de genou, comportant à la manière connue, un élément fémoral et un plateau tibial. L'élément fémoral est utilisé pour remplacer les condyles détruits du fémur, tandis que le plateau tibial se substitue à la partie supérieure endommagée du tibia.

Il existe déjà des prothèses de ce genre qui sont tout d'abord des prothèses à glissement, avec ou sans conservation du ligament postérieur croisé, dans laquelle l'élément fémoral glisse sur une zone de faible surface par rapport au plateau tibial.

Les caractéristiques du préambule de la revendication 1 sont connues du document US-A-5358527.

On connaît par le document GB-A-2 067 412, une prothèse de genou dont l'élément fémoral et pourvu entre ses deux condyles d'un passage se terminant par une came convexe. Le plateau tibial, de la prothèse considérée, comporte des empreintes de réception des condyles et d'une rampe concave s'élevant vers l'élément fémoral et pénétrant largement entre ses deux condyles.

Lors de la coopération de la came avec la rampe et après un déplacement angulaire libre, il se crée des contraintes considérables, du fait du bras de levier important, créé par rapport à la fixation du plateau tibial sur la partie supérieure du tibia.

De plus, l'importance de la pénétration de la rampe du plateau tibial limite considérablement l'amplitude de la rotation du tibia par rapport au fémur.

Enfin la hauteur de la rampe du plateau tibial entraîne une résection importante de l'épiphyse du fémur, et qui bien entendu diminue considérablement sa résistance.

Ces inconvénients ont été résolus dans le brevet européen N° 0 294 298 appartenant au demandeur. La prothèse totale de genou décrite dans ce document, comprend un élément fémoral muni de deux condyles asymétriques et d'une came convexe qui est disposée entre les deux condyles et réalisée sous la forme d'un troisième condyle.

Le plateau tibial comporte une piste horizontale recevant la came convexe de l'élément fémoral qui est situé en dessous du niveau des empreintes sur lesquelles glissent les condyles de l'élément fémoral. Le plateau tibial comprend, entre les empreintes et dans l'alignement de la piste, une rampe concave affectant la forme d'une butée courbe qui permet que le point de contact de la came se trouve à une faible distance du plateau tibial sur toute la course de contact de ces deux éléments, de manière à diminuer au maximum les contraintes sur ledit plateau tibial.

Du fait de la position basse de la came ou troisième condyle, on diminue au maximum le bras de levier créé par son action contre la rampe ou butée du plateau tibial, de sorte que les contraintes transmises au tibia sont minimes.

Les prothèses totales de genou décrites ci-dessus comportent certains inconvénients en ce qui concerne l'impossibilité de conserver le ligament croisé postérieur du fait de l'emplacement central de la came convexe entre les deux condyles.

En effet, le fémur d'un genou sain est composé de deux condyles distaux /postérieurs qui sont séparés par un espace permettant le passage du ligament croisé postérieur stabilisant l'articulation en position postérieure.

Les perfectionnements apportés à la prothèse de genou suivant la présente invention ont pour objet de conserver le passage du ligament croisé postérieur en gardant les avantages de la came convexe décrite dans le brevet européen N° 0 294 298.

La prothèse conforme à l'invention est définie dans la revendication 1.

La prothèse totale de genou suivant la présente invention comprend un élément fémoral pourvu de deux condyles, un externe, un interne, dont l'un au moins est solidaire sur sa côté externe d'une came convexe assurant la stabilisation postérieure et un plateau tibial comportant deux empreintes propres à recevoir lesdits condyles et au moins une piste disposée à proximité immédiate de l'empreinte recevant le condyle solidaire de la came convexe, ladite piste étant délimitée par au moins une butée courbe et au moins une empreinte horizontale 19, 24 entre les extrémités postérieures et antérieures du plateau tibial, tandis qu'entre les deux condyles de l'élément fémoral et les deux empreintes de plateau tibial sont prévus respectivement un espace libre et une échancrure pour le passage du ligament croisé postérieur du genou.

La prothèse totale de genou comprend sur le côté externe d'un condyle de l'élément fémoral, une came convexe réalisée sous la forme d'un troisième condyle de manière que l'espace libre soit délimité entre le condyle externe et le condyle interne pour communiquer avec l'échancrure du plateau tibial pour le passage du ligament croisé postérieur du genou, tandis que la came convexe coopère avec une piste située à l'extérieur et en dessous du niveau de l'empreinte recevant le condyle.

La prothèse totale de genou suivant à une mode de réalisation particulière la présente invention comprend un plateau tibial pourvu d'au moins une piste recevant la came convexe correspondante de l'élément fémoral et qui comporte du côté de l'extrémité postérieure du plateau une empreinte horizontale située en dessous du niveau des empreintes propres à recevoir le condyle, ladite empreinte se prolongeant vers l'extrémité antérieure dudit plateau par une butée courbe qui se dresse à l'opposé du tibia.

La prothèse totale de genou suivant à une mode de réalisation particulière la présente invention comprend un plateau tibial pourvu d'au moins une piste recevant la came convexe correspondante de l'élément fémoral et qui comporte du côté de l'extrémité postérieure du plateau une première butée courbe s'élevant légèrement au-dessus des empreintes prévues pour les condyles, ladite première butée se prolongeant vers l'extrémité antérieure dudit plateau par une seconde butée courbe qui se dresse à l'opposé du tibia.

La prothèse totale de genou suivant à une mode de réalisation particulière la présente invention comprend un plateau tibial pourvu d'au moins une piste recevant la came convexe correspondante de l'élément fémoral et qui comporte du côté de l'extrémité postérieure du plateau une butée courbe s'élevant légèrement au-dessus des empreintes prévues pour les condyles, ladite butée se prolongeant vers l'extrémité antérieure dudit plateau par une empreinte horizontale située en dessous du niveau desdites empreintes.

La prothèse totale de genou suivant à une mode de réalisation particulière la présente invention comprend une came qui présente un profil courbe s'étendant à partir du voile jusqu'à l'aile antérieure de l'élément fémoral.

La prothèse totale de genou suivant à une mode de réalisation particulière la présente invention comprend une came qui présente un profil en portion de tore.

La prothèse totale de genou suivant à une mode de réalisation particulière la présente invention comprend une came qui présente une largeur qui est inférieure à celle prévue pour les condyles externe et interne.

La description qui va suivre en regard des dessins annexés, donnés à titre d'exemples non limitatifs, permettra de mieux comprendre l'invention, les caractéristiques qu'elle présente et les avantages qu'elle est susceptible de procurer:
Figure 1 est une vue illustrant la prothèse totale de genou suivant la présente invention.
Figures 2 et 3 sont des vues montrant des variantes de la prothèse de genou suivant la présente invention.
Figure 4 est une vue représentant le glissement de l'élément fémoral sur le plateau tibial de la prothèse totale de genou suivant la présente invention.
Figures 5 et 6 sont des vues illustrant des variantes de plateau tibial pour le glissement de l'élément fémoral de la prothèse totale de genou suivant la présente invention. La figure 5 ne montre pas une variante de la prothèse totale de genou suivant la présente invention.

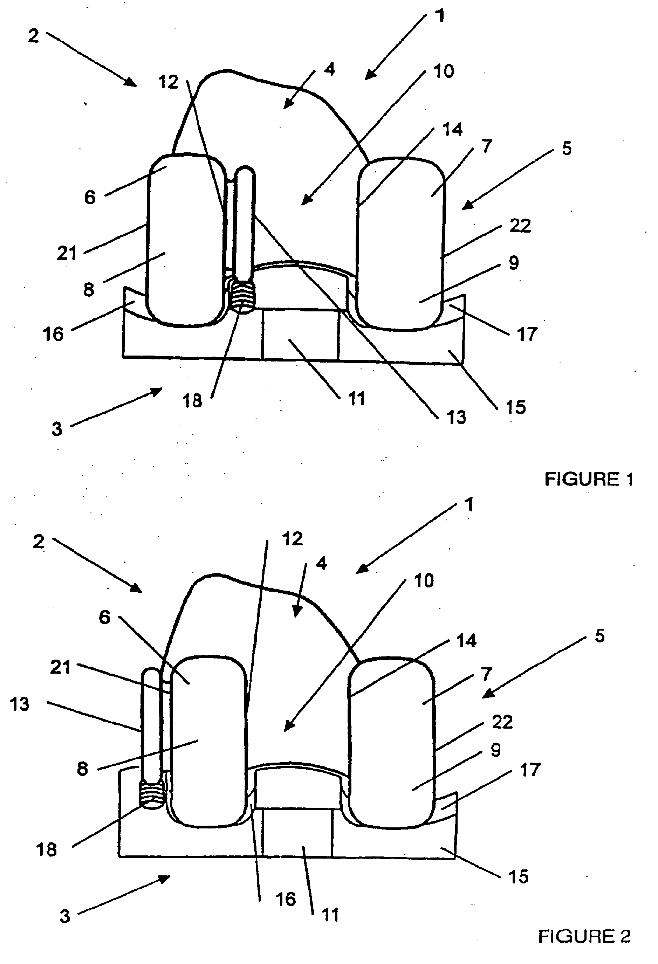

En figure 1 on a montré une prothèse totale du genou 1 qui comprend essentiellement un élément fémoral 2 et un plateau tibial 3.

L'élément fémoral 2 comporte en section transversale la forme générale d'un U, c'est à dire qu'il est formé d'une première aile antérieure 4 se prolongeant par une seconde aile postérieure 5.

Les faces internes planes de ses deux ailes sont destinées à coopérer avec des méplats ménagés au moyen de découpes antérieure et postérieure réalisées sur l'épiphyse du fémur non représenté.

L'aile postérieure 5 est constituée de deux voiles distincts et parallèles 6 et 7 solidaires du voile antérieure 4. La face externe des voiles 6 et 7 et de l'aile antérieure 4 présente un profil courbe définissant deux condyles latéraux 8 et 9 qui peuvent être symétrique ou asymétrique suivant l'utilisation de la prothèse 1.

Le condyle externe 8 est séparé du condyle interne 9 par un espace libre 10 qui communique avec un espace ou une échancrure 11 ménagée dans la partie postérieure du plateau tibial 3 pour le passage du ligament croisé postérieur non représenté.

Le condyle externe 8 comporte sur son côté interne 12 et dans l'espace libre 10 une came convexe 13 formant un troisième condyle. La came 13 présente un profil courbe s'étendant à partir du voile 6 jusqu'à l'aile antérieure 4.

La came convexe 13 solidaire du condyle externe 8 délimite avec le côté opposé et interne 14 du condyle interne 9 les dimensions de l'espace libre 10 prévu pour le passage du ligament croisé.

Le plateau tibial 3 est constitué d'un élément en matière plastique 15 à très faible coefficient de frottement destiné à être engagé sur une plaque métallique solidaire d'une queue, non représenté, qui vient s'ancrer dans le tibia.

L'élément 15 est creusé de part et d'autre de l'échancrure 11 de deux empreintes 16 et 17 recevant respectivement les condyles 8 et 9 de l'élément fémoral 2 pour permettre une rotation glissement du genou artificiel 1.

Entre les deux empreintes 16 et 17 et à proximité de celle 16, est ménagé une piste 18 destinée à recevoir la came convexe 13 solidaire du condyle externe 8.

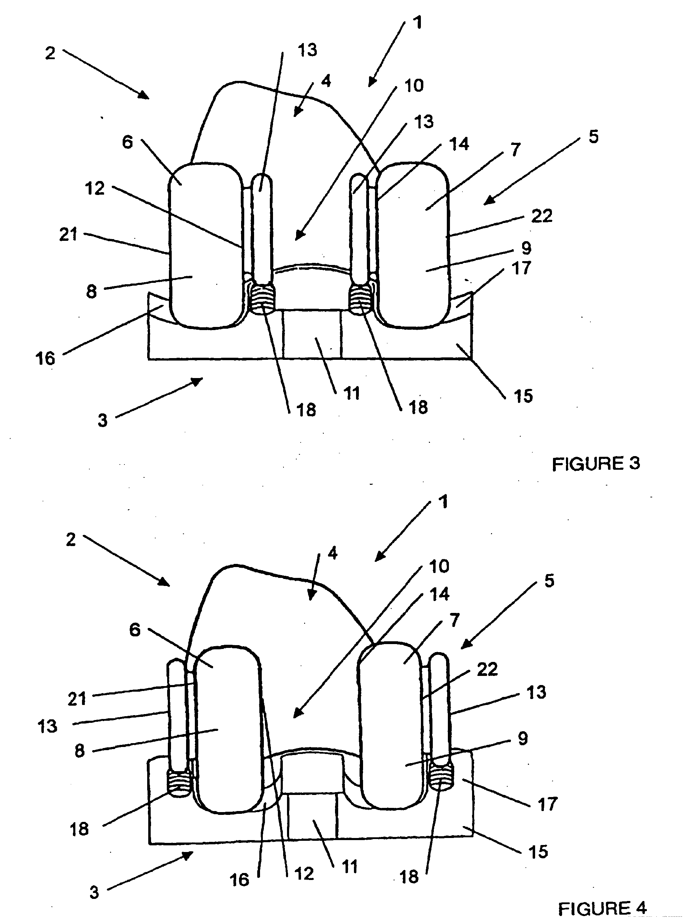

Le profil de la piste 18 peut varier en fonction de l'utilisation de la prothèse totale du genou 1, comme cela est montré en figures 4 à 6.

De la même manière que pour le condyle externe 8, le condyle interne 9 peut être solidaire sur son côté interne 14 d'une came 13 coopérant avec une piste 18 ménagée entre les deux empreintes 16 et 17, et à proximité de celle 17.

La came convexe 13, solidaire du côté interne 12 du condyle 8, permet de favoriser le mouvement anatomique de la prothèse 1, puisque le condyle externe 8 recule plus que l'autre par rapport au plateau tibial 3.

En figure 2 on a représenté le condyle externe 8 de l'élément fémoral 2 qui est solidaire de la came convexe 13 sur son côté externe 21, tandis que le plateau tibial 3 présente une piste 18 située à l'extérieur de l'empreinte 16. Dans cet exemple de réalisation l'espace 10 est compris entre les deux condyles 8 et 9 de l'élément fémoral 2 pour le passage du ligament croisé postérieur.

De la même manière que pour le condyle externe 8, le condyle interne 9 peut être solidaire sur son côté externe 22 d'une came convexe 13, tandis que le plateau tibial 3 présente une piste 18 située à l'extérieur de l'empreinte 17.

En figure 3 on a représenté une autre variante de la prothèse totale de genou 1 dont l'élément fémoral 2 comporte sur les côtés internes 12 et 14 de chaque condyle 8 et 9 une came convexe 13. Ainsi les deux cames convexes opposées 13 délimitent les dimensions de l'espace libre 10 pour le passage du ligament croisé postérieur. Chaque came 13 de l'élément fémoral 2 coopère avec une piste 18 ménagée entre et à proximité des empreintes 16 et 17 du plateau tibial 3 propre à recevoir les condyles 8 et 9.

La came convexe 13 représenté en figures 1 à 3, solidaire, soit du condyle externe 8, soit du condyle interne 9, soit des deux condyles 8 et 9, présente un profil en portion de tore permettant un petit jeu en rotation et évitant le phénomène de coupe.

On entend par un profil en portion de tore une surface de révolution engendrée par un cercle qui tourne autour d'un axe situé dans son plan et ne passant pas par son centre.

On note que la came convexe 13 peut être de manière plus générale une surface torique.

On entend par surface torique la surface engendrée par un cercle se déplaçant sur une ligne quelconque, le plan du cercle restant perpendiculaire à la ligne de déplacement.

Lorsque l'élément fémoral 2 comporte sur les côtés internes 12 et 14 de chaque condyle 8 et 9, une came 13, on constate que ce système ne change pas les appuis habituels du genou (figure 3).

De plus, l'élément fémoral 2 suivant l'invention, comportant une ou deux cames 13, permet un jeu de rotation par rapport à l'axe tibial.

En outre, l'élément fémoral 2 suivant l'invention, comportant une ou deux cames 13, permet de supprimer le conflit rotulien des prothèses de genou de l'art antérieur.

En effet, on remarque que la came convexe 13, vue de face, présente une largeur très faible, arrondie qui est très inférieure à celle prévue pour les condyles externe 8 et interne 9.

En fonction de la conservation ou non du ligament croisé postérieur la piste 18 recevant la came convexe 13 peut présenter des profils différents.

En effet, en figure 4 on a montré que la piste 18 comporte du côté de l'extrémité postérieure du plateau 3, une empreinte horizontale 19 se prolongeant vers l'extrémité antérieure dudit plateau par une butée courbe 20 qui se dresse à l'opposé du tibia en direction de l'élément fémoral 2. L'empreinte horizontale 19 est située en dessous des empreintes 16 et 17 recevant les condyles 8 et 9 de l'élément fémoral 2 (figure 4).

Le plateau tibial 3 peut être utilisé avec un élément fémoral 2 décrit en figure 1 à 3 pour constitué une prothèse totale du genou 1 avec conservation ou non du ligament croisé postérieur.

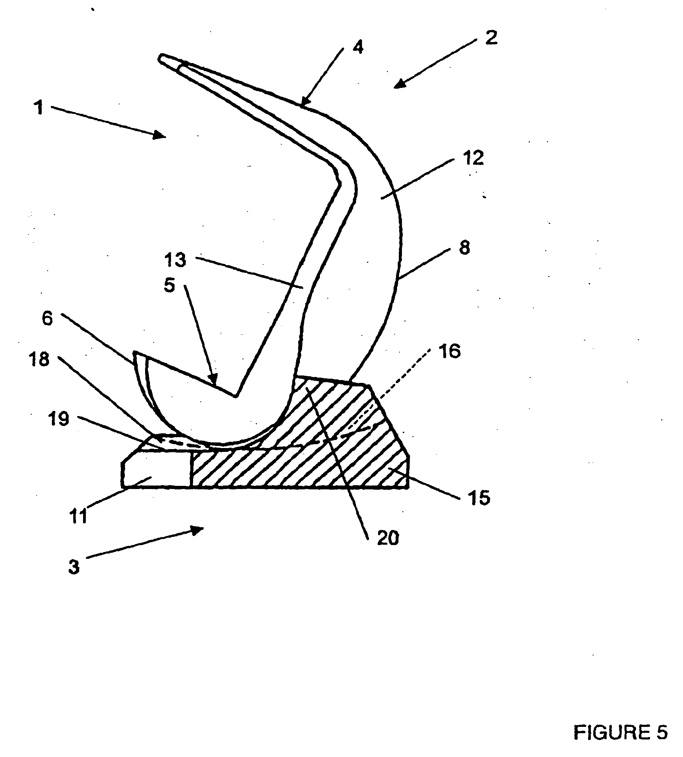

En figure 5 la piste 18 comporte du côté de l'extrémité postérieure du plateau tibial 3, c'est à dire du côté de l'échancrure 11, une première butée courbe 23 s'élevant légèrement au-dessus des empreintes 16 et 17 prévus pour les condyles 8 et 9. La première butée 23 se prolonge vers l'extrémité antérieure dudit plateau par une seconde butée courbe 20 qui se dresse à l'opposé du tibia.

C'est pourquoi la figure 5 ne montre pas une variante de la prothèse totale du genou suivant la présente invention.

Le plateau tibial 3 proposé en figure 5 peut être utilisé avec un élément fémoral 2 décrit en figure 1 à 3 pour permettre la conservation de ligaments postérieurs croisés, avec un passage automatique en fonctionnement postéro-stabilisé si le ligament vient à se détruire ultérieurement.

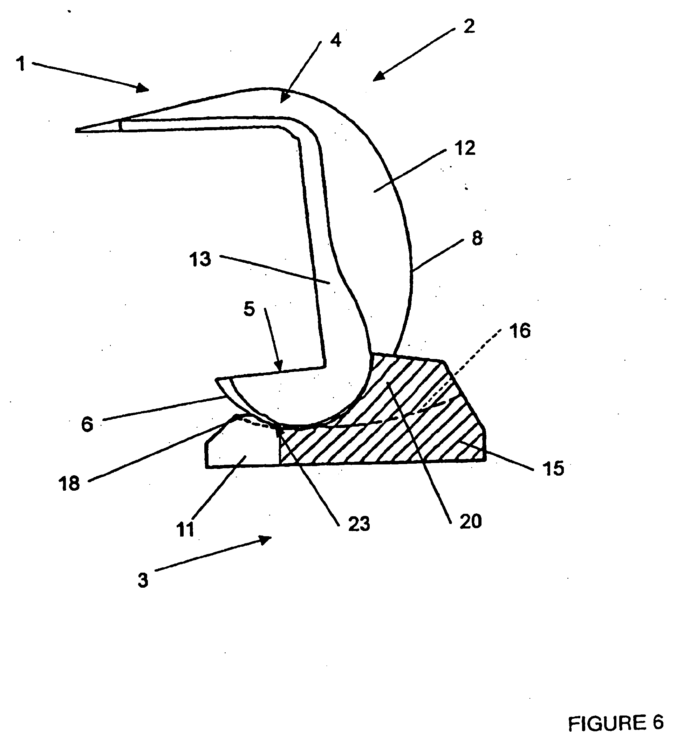

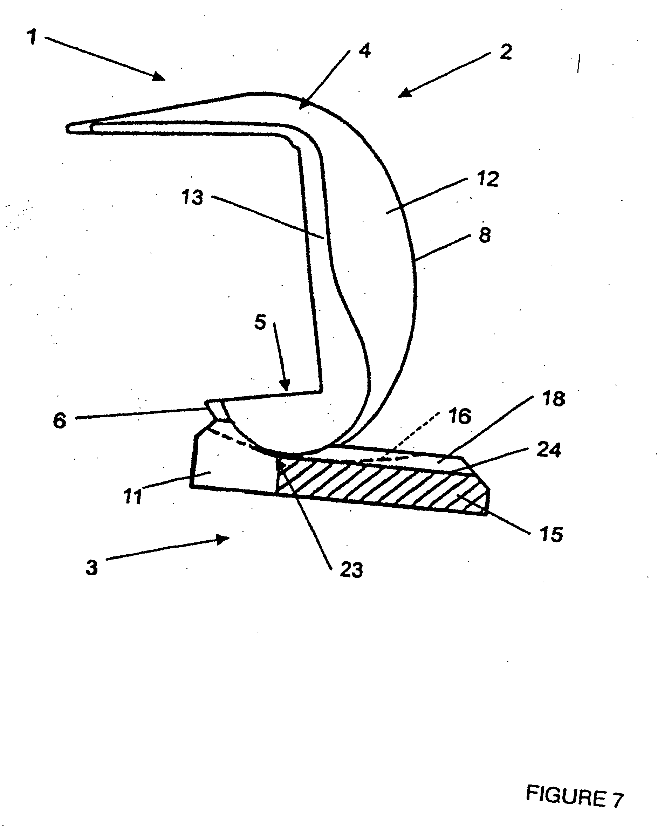

En figure 6 la piste 18 comporte du côté de l'extrémité postérieure du plateau tibial 3, c'est à dire au niveau de l'échancrure 11, la butée courbe 23 s'élevant légèrement au-dessus des empreintes 16 et 17, et se prolongeant vers l'extrémité antérieure dudit plateau par une empreinte horizontale 24 située en dessous desdites empreintes 16 et 17 pour les condyles 8 et 9 de l'élément fémoral 2.

Ainsi ce plateau tibial 3 peut être utilisé avec un élément fémoral 2 décrit précédemment en figures 1 à 3 pour constituer une prothèse totale du genou 1 avec la conservation de ligament croisé postérieur où le recul est maîtrisé par la butée 23 lors des déplacements de l'élément fémoral.

Il doit d'ailleurs être entendu que la description qui précède n'a été donnée qu'a titre d'exemple et qu'elle ne limite nullement le domaine de l'invention dont on ne sortirait pas en remplaçant les détails d'exécution décrits par tout autre équivalent.

## Revendications

1. Prothèse totale de genou comprenant :
• Un élément fémoral (2) comportant deux condyles (8, 9), un externe, un interne et au moins une came convexe (13) solidaire des condyles (8, 9) pour assurer la stabilisation postérieure de la prothèse,
• Un plateau tibial (3) muni d'empreintes (16, 17) propre à recevoir les condyles (8, 9) et d'au moins une piste (18) disposée à proximité immédiate desdites empreintes afin de coopérer avec la came convexe (13),
• Un espace libre (10) et une échancrure (11) ménagés respectivement entre les condyles (8, 9) de l'élément fémoral (2) et les empreintes (16, 17) du plateau tibial (3) pour le passage du ligament croisé postérieur du genou, **caractérisée en ce que** l'un au moins des côtés externes (21, 22) des condyles (8, 9) est solidaire d'une came convexe (13) réalisée sous la forme d'un troisième condyle et coopérant avec une piste (18) qui est délimitée par une butée courbe (20, 23) et au moins une empreinte horizontale (19, 24) entre les extrémités postérieures et antérieures du plateau tibial (3).

2. Prothèse totale du genou suivant la revendication 1, **caractérisée en ce que** le côté externe (21, 22) d'un condyle (8, 9) de l'élément fémoral (2) est solidaire d'une came convexe (13) réalisée sous la forme d'un troisième condyle de manière que l'espace libre (10) soit délimité entre les deux condyles (8, 9) pour communiquer avec l'échancrure (11) du plateau tibial (3) pour le passage du ligament croisé postérieur du genou, tandis que la came convexe (13) coopère avec une piste (18) située à l'extérieur et en dessous du niveau de l'empreinte (16, 17) recevant le condyle (8, 9).

3. Prothèse totale du genou suivant la revendication 1, **caractérisée en ce que** le plateau tibial (3) est pourvu d'au moins une piste (18) recevant la came convexe (13) correspondante de l'élément fémoral (2) et qui comporte du côté de l'extrémité postérieure dudit plateau une empreinte horizontale (19) située en dessous du niveau des empreintes (16, 17) propres à recevoir les condyles (8, 9), ladite empreinte (19) se prolongeant vers l'extrémité antérieure dudit plateau par une butée courbe (20) qui se dresse à l'opposé du tibia.

4. Prothèse totale du genou suivant la revendication 1, **caractérisée en ce que** le plateau tibial (3) est pourvu d'au moins une piste (18) recevant la came convexe (13) correspondante de l'élément fémoral (2) et qui comporte du côté de l'extrémité postérieure dudit plateau une première butée courbe (23) s'élevant légèrement au-dessus des empreintes (16, 17) propre à recevoir les condyles (8, 9), ladite première butée se prolongeant vers l'extrémité antérieure dudit plateau par une seconde butée courbe (20) qui se dresse à l'opposé du tibia.

5. Prothèse totale du genou suivant la revendication 1, **caractérisée en ce que** le plateau tibial (3) est pourvu d'au moins une piste (18) recevant la came convexe (13) correspondante de l'élément fémoral (2) et qui comporte, du côté de l'extrémité postérieure dudit plateau, une butée courbe (23) s'élevant légèrement au-dessus des empreintes (14, 17) propres à recevoir les condyles (8, 9), ladite butée se prolongeant vers l'extrémité antérieure dudit plateau par une empreinte horizontale (24) située en dessous du niveau desdites empreintes (16, 17).

6. Prothèse totale du genou suivant la revendication 1, **caractérisée en ce que** la came (13) présente un profil courbe s'étendant à partir du voile (6) jusqu'à l'aile antérieure (4) de l'élément fémoral (2).

7. Prothèse totale de genou suivant la revendication 1, **caractérisée en ce que** la came (13) présente un profil en portion de tore.

8. Prothèse totale de genou suivant la revendication 1, **caractérisée en ce que** la came (13) est une surface torique.

9. Prothèse totale de genou suivant la revendication 1, **caractérisée en ce que** la came (13) présente une largeur qui est inférieure à celle prévue pour les condyles externe (8) et interne (9).

## Claims

1. Total knee prosthesis comprising:
• A femoral element (2) with two condyles (8, 9), one external and one internal, and at least one convex cam (13) integral with the condyles (8, 9) to provide posterior stabilisation of the prosthesis,
• A tibial plate (3) provided with recesses (16, 17) to take the condyles (8, 9) and at least one track (18) arranged right next to these recesses in order to work with the convex cam (13),
• A free space (10) and an indentation (11) arranged between the condyles (8, 9) of the femoral element (2) and the recesses (16, 17) of the tibial plate (3) respectively for the posterior cruciate ligament of the knee to pass through,
**characterised in that** at least one of the external sides (21, 22) of the condyles (8, 9) is integral with a convex cam (13) made in the form of a third condyle working with a track (18), which is delimited by a curved stop (20, 23), and at least one horizontal recess (19, 24) between the front and rear ends of the tibial plate (3).

2. Total knee prosthesis according to claim 1, **characterised in that** the external side (21, 22) of a condyle (8, 9) of the femoral element (2) is integral with a convex cam (13) made in the form of a third condyle so that the empty space (10) is delimited between the two condyles (8, 9) to communicate with the indentation (11) of the tibial plate (3) for the posterior cruciate ligament of the knee to pass through, whereas the convex cam (13) works with a track (18) situated outside below the level of the recess (16, 17) taking the condyle (8, 9).

3. Total knee prosthesis according to claim 1, **characterised in that** the tibial plate (3) is provided with at least one track (18) taking the convex cam (13) corresponding to the femoral element (2), which on the side of the rear end of this plate has a horizontal recess (19) situated below the level of the recesses (16, 17) to take the condyles (8, 9), this recess (19) extending towards the front end of this plate by a curved stop (20), which is set up opposite the tibia.

4. Total knee prosthesis according to claim 1, **characterised in that** the tibial plate (3) is provided with at least one track (18) taking the convex cam (13) corresponding to the femoral element (2), which on the side of the rear end of this plate has a first curved stop (23) rising slightly above the recesses (16, 17) to take the condyles (8, 9), this first stop extending towards the front end of this plate by a second curved stop (20), which is set up opposite the tibia.

5. Total knee prosthesis according to claim 1, **characterised in that** the tibial plate (3) is provided with at least one track (18) taking the convex cam (13) corresponding to the femoral element (2), which on the side of the rear end of this plate has a curved stop (23) rising slightly above the recesses (14, 17) to take the condyles (8, 9), this stop extending towards the front end of this plate by a horizontal recess (24) situated below the level of these recesses (16, 17).

6. Total knee prosthesis according to claim 1, **characterised in that** the cam (13) has a curved profile extending from the sheet (6) as far as the front wing (4) of the femoral element (2).

7. Total knee prosthesis according to claim 1, **characterised in that** the cam (13) has the profile of a part of a core.

8. Total knee prostheses according to claim 1, **characterised in that** the cam (13) is the surface of a core.

9. Total knee prosthesis according to claim 1, **characterised in that** the cam (13) has a width, which is less than that provided for the external (8) and internal (9) condyles.

## Patentansprüche

1. Knie-Totalprothese, die enthält:
• ein Femurteil (2), das zwei Kondyle (8, 9), einen äußeren und einen inneren, und mindestens eine fest mit den Kondylen (8, 9) verbundene, konvexe Nocke (13) aufweist, um die posteriore Stabilisierung der Prothese zu gewährleisten,
• ein Tibiaplateau (3), das mit zur Aufnahme der Kondyle (8, 9) geeigneten Mulden (16, 17) und mit mindestens einer Bahn (18) versehen ist, die in direkter Nähe der Mulden angeordnet ist, um mit der konvexen Nocke (13) zusammenzuwirken,
• einen Freiraum (10) und eine Aussparung (11), die zwischen den Kondylen (8, 9) des Femurteils (2) bzw. den Mulden (16, 17) des Tibiaplateaus (3) für den Durchgang des hinteren Kreuzbands des Knies angeordnet sind,
**dadurch gekennzeichnet, dass** mindestens eine der Außenseiten (21, 22) der Kondyle (8, 9) fest mit einer konvexen Nocke (13) verbunden ist, die in Form eines dritten Kondyls hergestellt ist und mit einer Bahn (18) zusammenwirkt, die von einem gekrümmten Anschlag (20, 23) und mindestens einer waagerechten Mulde (19, 24) zwischen den posterioren und anterioren Enden des Tibiaplateaus (3) begrenzt wird.

2. Knie-Totalprothese nach Anspruch 1, **dadurch gekennzeichnet, dass** die Außenseite (21, 22) eines Kondyls (8, 9) des Femurteils (2) fest mit einer konvexen Nocke (13) verbunden ist, die in Form eines dritten Kondyls so hergestellt ist, dass der Freiraum (10) zwischen den zwei Kondylen (8, 9) begrenzt wird, um mit der Aussparung (11) des Tibiaplateaus (3) für den Durchgang des hinteren Kreuzbands des Knies in Verbindung zu stehen, während die konvexe Nocke (13) mit einer Bahn (18) zusammenwirkt, die sich außer- und unterhalb der Ebene der den Kondyl (8, 9) aufnehmenden Mulde (16, 17) befindet.

3. Knie-Totalprothese nach Anspruch 1, **dadurch gekennzeichnet, dass** das Tibiaplateau (3) mit mindestens einer Bahn (18) versehen ist, die die entsprechende konvexe Nocke (13) des Femurteils (2) aufnimmt, und die auf der Seite des posterioren Endes des Plateaus eine waagerechte Mulde (19) aufweist, die sich unter der Ebene der zur Aufnahme der Kondyle (8, 9) geeigneten Mulden (16, 17) befindet, wobei die Mulde (19) sich zum anterioren Ende des Plateaus durch einen gekrümmten Anschlag (20) verlängert, der sich entgegengesetzt zum Schienbein erhebt.

4. Knie-Totalprothese nach Anspruch 1, **dadurch gekennzeichnet, dass** das Tibiaplateau (3) mit mindestens einer Bahn (18) versehen ist, die die entsprechende konvexe Nocke (13) des Femurteils (2) aufnimmt, und die auf der Seite des posterioren Endes des Plateaus einen ersten gekrümmten Anschlag (23) aufweist, der sich leicht oberhalb der zur Aufnahme der Kondyle (8, 9) geeigneten Mulden (16, 17) erhebt, wobei der erste Anschlag sich zum anterioren Ende des Plateaus durch einen zweiten gekrümmten Anschlag (20) verlängert, der entgegengesetzt zum Schienbein hochragt.

5. Knie-Totalprothese nach Anspruch 1, **dadurch gekennzeichnet, dass** das Tibiaplateau (3) mit mindestens einer Bahn (18) versehen ist, die die entsprechende konvexe Nocke (13) des Femurteils (2) aufnimmt, und die auf der Seite des posterioren Endes des Plateaus einen gekrümmten Anschlag (23) aufweist, der sich leicht oberhalb der zur Aufnahme der Kondyle (8, 9) geeigneten Mulden (14, 17) erhebt, wobei der Anschlag sich zum anterioren Ende des Plateaus durch eine waagerechte Mulde (24) verlängert, die sich unter der Ebene der Mulden (16, 17) befindet.

6. Knie-Totalprothese nach Anspruch 1, **dadurch gekennzeichnet, dass** die Nocke (13) ein gekrümmtes Profil aufweist, das sich ausgehend von der Schale (6) bis zum anterioren Flügel (4) des Femurteils (2) erstreckt.

7. Knie-Totalprothese nach Anspruch 1, **dadurch gekennzeichnet, dass** die Nocke (13) ein Profil in Form eines Torusabschnitts aufweist.

8. Knie-Totalprothese nach Anspruch 1, **dadurch gekennzeichnet, dass** die Nocke (13) eine torusförmige Fläche ist.

9. Knie-Totalprothese nach Anspruch 1, **dadurch gekennzeichnet, dass** die Nocke (13) eine Breite aufweist, die geringer als diejenige ist, die für den äußeren (8) und inneren Kondyl (9) vorgesehen ist.
